# EUROPÄISCHE PATENTANMELDUNG

(11) **EP 0 523 417 A1**
(43) Veröffentlichungstag der Anmeldung: **20.01.1993**
(21) Anmeldenummer: 92110701.7
(22) Anmeldetag: 25.06.1992
(51) Int. Cl.: A61N 5/10

(54) **Rohrkanalweiche**

(30) Priorität: 16.07.1991 DE 4123501
(71) Anmelder: ISOTOPEN-TECHNIK DR. SAUERWEIN GMBH, D-42781 Haan (DE)
(72) Erfinder: Link, Rainer, Dr., W-5014 Kerpen-Horrem (DE)
(74) Vertreter: König, Reimar, Dr.-Ing.

(57) **Zusammenfassung**

Rohrkanalweiche zum Verteilen von an Transportkabeln angeordneten Strahlern und/oder Prüfkörpern für die Strahlentherapie mit einem ortsfesten Verteiler (1, 9) und mindestens einem, gegebenenfalls lösbar befestigten, mit einem Applikator (3) verbundenen Kabelschlauch (2), wenigstens zwei beweglichen Weichenelementen (4, 10) mit wenigstens je einem Weichenschlauch (5) oder wenigstens einem beweglichen Weichenelement mit mindestens zwei Weichenschläuchen (5) und einem Antrieb (6, 7, 11) für jedes bewegliche Weichenelement.

## Beschreibung

Die Erfindung betrifft eine Rohrkanalweiche zum Verteilen von an Transportkabeln angeordneten Strahlern und/oder Prüfkörpern für die Strahlentherapie.

Eine derartige Rohrkanalweiche ist in der deutschen Patentschrift 33 13 857 beschrieben. Bei dieser Rohrkanalweiche sind an einem feststehenden Verteiler mehrere mit Applikatoren verbundene Kabelschläuche lösbar befestigt. Parallel zum Verteiler ist eine drehbare Scheibe angeordnet, die mit einem Weichenkanal verbunden ist. Die drehbare Scheibe läßt sich durch einen Antrieb schrittweise drehen, so daß sich der Ausgang des Weichenkanals fluchtend zum Eingang eines Kabelschlauchs bewegen läßt. Zu diesem Zweck sind die Kabelschläuche auf einem zur Drehachse der Scheibe konzentrischen Kreis angeordnet. Der Weichenkanal verläuft von der drehbaren Scheibe in einem Bogen in die Nähe der Drehachse und ist dort mit dem Ausgang eines Abschirmkörpers drehbar verbunden. Ein Antrieb für das Transportkabel mit dem Strahler erlaubt es, den Strahler aus dem Abschirmkörper durch den Weichenkanal in einen damit verbundenen Kabelschlauch bis in einen Applikator zu verfahren. Um sicherzustellen, daß kein Strahler ins Freie gelangt, wenn der Weichenkanal vor einem Ausgang ohne angeschlossenen Kabelschlauch steht, sind Sperren im Bereich des Verteilers und der drehbaren Scheibe angeordnet, die ein Verfahren des Strahlers verhindern, wenn die drehbare Scheibe mit dem Weichenkanal nicht richtig positioniert ist oder vor einem Ausgang ohne Kabelschlauch steht.

Mit dieser Rohrkanalweiche läßt sich ein Strahler nacheinander zu mehreren Applikatoren verfahren, um eine Strahlentherapie durchzuführen. Ein Prüfen der Applikatoren und Kabelschläuche mittels eines Prüfkörpers läßt sich nur durchführen, wenn zuvor der Abschirmkörper mit dem Antrieb für das Transportkabel mit dem Strahler von der Rohrkanalweiche gelöst und durch einen anderen Antrieb für ein Transportkabel mit einem Prüfkörper ersetzt wird. Es liegt auf der Hand, daß eine solche Konstruktion aufwendig ist und für das Umrüsten Zeit erfordert.

In der europäischen Patentschrift 0 254 351 ist eine Vorrichtung beschrieben, mit der sich wahlweise ein Prüfkörper oder ein Strahler in einen Applikator bewegen läßt. Zu diesem Zweck sind zwei Antriebe für je ein Transportkabel mit einem Strahler bzw. einem Prüfkörper parallel zueinander angeordnet. Die beiden zugehörigen Kabelschläuche werden in einer festen Zwei-Kanal-Weiche zusammengeführt, von wo aus ein einziger Kabelschlauch zu einem Applikator führt. Mit dieser Vorrichtung läßt sich zunächst ein Prüfkörper bis in den Applikator verfahren, um festzustellen, ob der Kabelschlauch bis zum Applikator so verläuft, daß der Prüfkörper und folglich später auch der Strahler behinderungsfrei bis in den Applikator gelangen können. Diese bekannte Vorrichtung läßt keine Flächentherapie mit mehreren Applikatoren zu, da sich an die feste Weiche nur ein Applikator anschließen läßt. Des weiteren lassen sich der Prüfkörper und der Strahler stets nur nacheinander in den Applikator verfahren.

Der Erfindung liegt daher die Aufgabe zugrunde, eine Rohrkanalweiche zu schaffen, mit der sich ein Applikator nacheinander mit einem Prüfkörper und einem Strahler beschicken läßt oder auch ein gleichzeitiges Beschicken mehrerer Applikatoren mit mehreren Strahlern und/oder Prüfkörpern gleichzeitig möglich ist.

Die Lösung dieser Aufgabe besteht darin, daß eine Rohrkanalweiche der eingangs erwähnten Art erfindungsgemäß einen feststehenden Verteiler mit mindestens zwei, mit mindestens einem Applikator verbundenen Kabelschläuchen, des weiteren wenigstens zwei bewegliche Weichenelemente mit mindestens je einem Weichenschlauch oder wenigstens ein bewegliches Weichenelement mit mindestens zwei Weichenschläuchen und einen Antrieb für jedes bewegliche Weichenelement aufweist.

Die erfindungsgemäße Rohrkanalweiche besteht somit im einfachsten Fall aus einem feststehenden Verteiler mit mindestens einem ggf. lösbar befestigten, mit einem Applikator verbundenen Kabelschlauch und wenigstens einem beweglichen Weichenelement mit zwei Weichenschläuchen. Der eine Weichenschlauch dient zum Führen eines Transportkabels mit einem Prüfkörper in dem Kabelschlauch bis zu dem damit verbundenen Applikator, während der andere Weichenschlauch dazu dient, das Transportkabel eines Strahlers in denselben Kabelschlauch zu führen. So kann nacheinander ein Applikator geprüft und für die Strahlentherapie eingesetzt werden.

Selbstverständlich lassen sich auch die beiden Weichenkanäle zum Beschicken zweier mit je einem Applikator verbundenen Kabelschläuchen mit Strahlern und/oder Prüfkörpern einsetzen, so daß sich die Applikatoren gleichzeitig mit zwei Strahlern, zwei Prüfkörpern oder einem Strahler und einem Prüfkörper beschicken lassen.

Vorzugsweise sind mit dem ortsfesten Verteiler eine größere Anzahl von Kabelschläuchen mit Applikatoren verbunden, während am beweglichen Weichenelement mindestens zwei Weichenschläuche angeordnet sind. Mit einer solchen Anordnung lassen sich die Applikatoren gruppenweise mit Strahlern beschicken und ebenso mittels Prüfkörpern überprüfen.

Bei wenigstens zwei beweglichen Weichenelementen mit mindestens je einem Weichenschlauch lassen sich die Weichenelemente unabhängig voneinander bewegen, so daß sich beispielsweise mit einem Weichenelement, das mehrere Weichenschläuche aufweist, mehrere Strahler zu einer Applikatorgruppe leiten lassen, während über ein weiteres bewegliches Weichenelement mit nur einem Weichenschlauch ein Prüfkörper nacheinander eine Gruppe von Kabelhüllen mit Applikatoren abfährt.

Das bewegliche Weichenelement kann als drehbare Scheibe gestaltet sein; die am feststehenden Verteiler angeschlossenen Kabelschläuche sind dann auf wenigstens einem zur Drehachse der drehbaren Scheibe konzentrischen Kreis angeordnet. Der Radius dieses konzentrischen Kreises entspricht dem Abstand der Weichenkanalmündungen in der drehbaren Scheibe von der Drehachse, so daß sich die Ausgänge der Weichenschläuche fluchtend zum Eingang des jeweils angefahrenen Kabelschlauchs ausrichten lassen.

Das bewegliche Weichenelement besteht jedoch vorzugsweise aus einem linearbeweglichen Schieber, wobei in diesem Fall die am feststehenden Verteiler angeordneten Kabelschläuche geradlinig parallel zueinander angeordnet sind.

Bei der erfindungsgemäßen Rohrkanalweiche lassen sich die aus der deutschen Patentschrift 33 13 857 bekannten Sicherungs-, Positionier- und Rastelemente verwenden. Auch der Antrieb der drehbaren Scheibe kann entsprechend gestaltet sein.

Die Erfindung wird nachstehend anhand zweier in der Zeichnung dargestellter Ausführungsbeispiele des näheren erläutert. In der Zeichnung zeigen
- Fig. 1:: eine schematische perspektivische, ausschnittsweise Darstellung einer Rohrkanalweiche mit drehbarem Weichenelement und
- Fig. 2:: eine schematische Darstellung einer Rohrkanalweiche mit einem Weichenschieber.

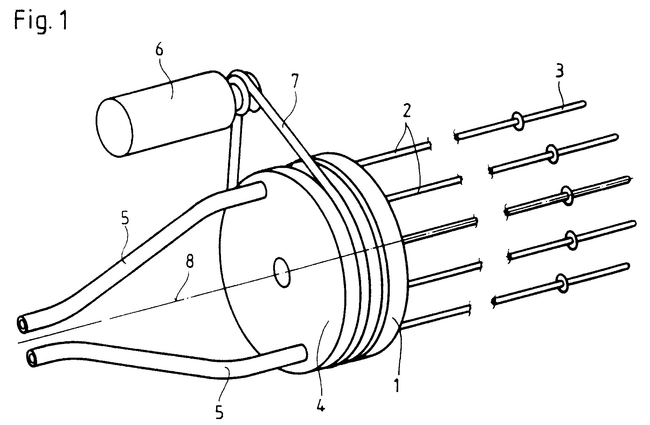

Die in Fig. 1 dargestellte Rohrkanalweiche zeigt nur die für die Erfindung wesentlichen Teile. Die sonst noch erforderlichen Bauteile entsprechen den in der deutschen Patentschrift 33 13 857 beschriebenen, auf die hinsichtlich der Offenbarung dieser Bauteile verwiesen wird.

An einem ortsfesten scheibenförmigen Verteiler 1 sind mehrere Kabelschläuche 2 mittels nicht sichtbarer Schlauchkupplungen lösbar befestigt. Die Schlauchkupplungen sind auf einem Kreis oder Kreisabschnitt angeordnet. Die Kabelhüllen 2 führen bis zu Applikatoren 3, die sich mit den Kabelhüllen 2 kuppeln lassen und intrakavitär oder oberflächlich an einem Patienten angeordnet sind.

Koaxial zur ortsfesten Verteilerscheibe 1 ist ein bewegliches Weichenelement in Form einer drehbaren Scheibe 4 angeordnet, das um eine Achse 8 drehbar ist. An der drehbaren Scheibe 4 sind zwei Weichenschläuche 5 befestigt, deren Abstand von der Drehachse 8 gleich dem Radius des Kreises ist, auf dem die Kupplungen für die Kabelschläuche 2 angeordnet sind. Auf diese Weise lassen sich die Mündungen der Kabelkanäle 5 wahlweise mit den Eingängen der Kabelhüllen 2 fluchtend verbinden. Die Weichenschläuche 5 sind mit ihren freien Enden in die Nähe der Drehachse 8 geführt und dort in nicht dargestellter Weise z. B. mit einem Abschirmkörper verbunden, so daß sich ein Strahler mittels eines Transportkabels aus dem Abschirmkörper heraus über einen Weichenschlauch 5 und einen der Kabelschläuche 2 bis in einen Applikator 3 verfahren läßt. Beide Weichenschläuche 5 können als Führung für je einen Strahler an einem Transportkabel dienen. Vorzugsweise dient ein Weichenschlauch 5 jedoch nur zum Führen eines Strahlers, während der andere Weichenschlauch 5 zum Führen eines Prüfkörpers dient. Auf diese Weise ist es möglich, über einen Weichenschlauch 5 einen Strahler bis in einen Applikator 3 zu führen, während gleichzeitig ein anderer Kabelschlauch 2 und ein Applikator 3 mittels des Prüfkörpers auf richtigen Verlauf und einwandfreie Verbindung geprüft werden.

Es lassen sich auch mehr als zwei Weichenschläuche an der drehbaren Scheibe 4 anordnen, so daß sich jeweils Gruppen von Applikatoren 3 mit Strahlern und einzelne oder Gruppen von Applikatoren 3 mit Prüfkörpern beschicken lassen. Die Mindestanzahl der mit dem Verteiler 1 verbundenen Kabelschläuche 2 und Applikatoren 3 beträgt zwei, vorzugsweise weist der Verteiler 1 jedoch eine größere Anzahl von Kabelschläuchen 2 mit Applikatoren 3 auf, als Weichenschläuche 5 an der drehbaren Scheibe 4 angeordnet sind.

Die drehbare Scheibe 4 läßt sich mittels eines Antriebsmotors 6 über einen Riemen 7 schrittweise in Drehung versetzen, so daß die Weichenschläuche 5 schrittweise zum Fluchten mit den Kabelschläuchen 2 kommen. Es ist auch möglich, zwei drehbare Scheiben 4 parallel zueinander mit je einem Antriebsmotor 6 und einem Riemen 7 anzuordnen und je einen Weichenschlauch 5 mit je einer drehbaren Scheibe 4 zu verbinden. Mit dieser Anordnung lassen sich die drehbaren Scheiben 4 mit den Weichenschläuchen 5 unabhängig voneinander bewegen, was dann von Vorteil ist, wenn sich ein Strahler in einem Applikator 3 länger aufhalten muß, als zum Testen der anderen Kabelschläuche 2 und Applikatoren 3 mittels eines Prüfkörpers erforderlich ist.

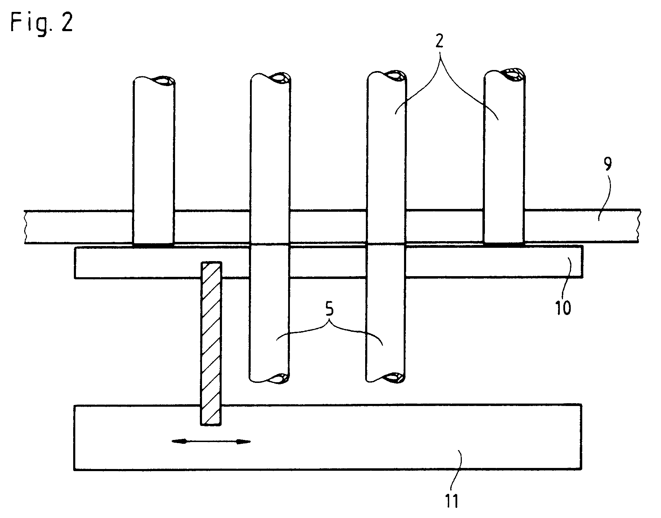

In Fig. 2 ist schematisch eine andere Ausführungsform der erfindungsgemäßen Weiche dargestellt, bei der der Verteiler 9 geradlinig parallel nebeneinander angeordnete Kabelschläuche 2 besitzt. Dementsprechend ist das bewegliche Weichenelement als Linearschieber 10 ausgebildet, an dem zwei oder mehr Weichenschläuche 5 angeordnet sein können. Ein Antrieb 11 ist mit dem Schieber 10 verbunden und bewirkt die Schieberbewegung derart, daß jeweils mindestens ein Kabelschlauch 2 mit mindestens einem Weichenkanal 5 in Verbindung steht. Dient der eine Weichenschlauch 5 zum Führen eines Prüfkörpers und der andere Weichenschlauch 5 zum Führen eines Strahlers, muß sich der Schieber 10 so weit bewegen lassen, daß sich auch die äußeren Kabelschläuche 2 sowohl mit einem Strahler als auch mit einem Prüfkörper beschicken lassen. In den Extremstellungen läßt sich nur ein Weichenschlauch 5 mit einem Kabelschlauch 2 verbinden, während in den Zwischenstellungen jeweils zwei Kabelschläuche 2 mit beiden Weichenschläuchen 5 gekuppelt sind.

Die Funktionsweise der in Fig. 2 dargestellten Weiche ist im übrigen gleich der in Fig. 1 dargestellten, und es lassen sich dieselben, in der deutschen Patentschrift 33 13 857 beschriebenen Sicherheits-, Positionier- und Rastvorrichtungen verwenden.

## Patentansprüche

1. Rohrkanalweiche zum Verteilen von an Transportkabeln angeordneten Strahlern und/oder Prüfkörpern für die Strahlentherapie mit
- einem feststehenden Verteiler (1, 9) und mindestens einem mit einem Applikator (3) verbundenen Kabelschlauch (2),
- wenigstens zwei beweglichen Weichenelementen (4, 10) mit mindestens je einem Weichenschlauch (5) oder wenigstens einem beweglichen Weichenelement mit mindestens zwei Weichenschläuchen (5).

2. Rohrkanalweiche nach Anspruch 1, dadurch gekennzeichnet, daß ein Weichenschlauch (5) mit einem Transportkabel mit Strahler und ein Weichenschlauch (5) mit einem Transportkabel mit Prüfkörper beaufschlagt ist.

3. Rohrkanalweiche nach Anspruch 1 oder 2, dadurch gekennzeichnet, daß am ortsfesten Verteiler (1, 9) mehr Kabelschläuche (2) als Weichenschläuche (5) angeordnet sind.

4. Rohrkanalweiche nach einem oder mehreren der Ansprüche 1 bis 3, dadurch gekennzeichnet, daß das bewegliche Weichenelement aus einer drehbaren Scheibe (4) besteht und die am ortsfesten Verteiler (1) angeordneten Kabelschläuche (2) auf wenigstens einem zur Drehachse (8) der drehbaren Scheibe (4) konzentrischen Kreis angeordnet sind.

5. Rohrkanalweiche nach einem oder mehreren der Ansprüche 1 bis 3, dadurch gekennzeichnet, daß das bewegliche Weichenelement aus einem linear beweglichen Schieber (10) besteht und die am ortsfesten Verteiler (9) angeordneten Kabelschläuche (2) geradlinig parallel zueinander angeordnet sind.

6. Rohrkanalweiche nach einem der Ansprüche 1 bis 5, gekennzeichnet durch einen Antrieb (6,7;11) für jedes bewegliche Weichenelement (4,10).
